(19) Europäisches Patentamt
European Patent Office
Office européen des brevets

(11) Veröffentlichungsnummer: **0 123 217**
**A1**

(12) # EUROPÄISCHE PATENTANMELDUNG

(21) Anmeldenummer: 84104094.2

(22) Anmeldetag: 12.04.84

(51) Int. Cl.³: **A 01 N 43/64**
A 61 K 31/53, C 07 D 251/34

(30) Priorität: 23.04.83 DE 3314739

(43) Veröffentlichungstag der Anmeldung: 31.10.84 Patentblatt 84/44

(84) Benannte Vertragsstaaten: AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Haberkorn, Axel, Dr.
Fuhlrottstrasse 99
D-5600 Wuppertal 1(DE)

(72) Erfinder: Kume, Toyohiko
6-7-8, Asahigaoka
Hino-Shi Tokyo(JP)

(54) **1-(4-(4-(Fluoralkylmethylthio- oder -sulfinyl-oder-sulfonyl-)phenoxy)phenyl)-1,3,5-triazin-2,4,6(1H,3H,5H)-trione, Verfahren zu ihrer Herstellung und ihre Verwendung als Coccidiosemittel.**

(57) Die Erfindung betrifft neue 1-{4-[4- (Fluoralkylmethylthio-, -sulfinyl- oder -sulfonyl-) phenoxy] phenyl}- 1,3,5-triazin -2,4,6 (1H,3H,5H) -trione, Verfahren zu ihrer Herstellung und ihre pharmazeutische Verwendung als Coccidiosemittel für den Menschen und verschiedene Arten von Tieren.

EP 0 123 217 A1

Croydon Printing Company Ltd.

BAYER AKTIENGESELLSCHAFT 5090 Leverkusen, Bayerwerk

Konzernverwaltung RP
Patentabteilung Ad/by-c

# 1- { 4-/4-(Fluoralkylmethylthio- oder -sulfinyl- oder -sulfonyl-)phenoxy7phenyl } -1,3,5-triazin-2,4,6(1H,3H, 5H)-trione, Verfahren zu ihrer Herstellung und ihre Verwendung als Coccidiosemittel

Die Erfindung betrifft neue 1-{4-/4-(Fluoralkylmethylthio-, -sulfinyl- oder -sulfonyl-)phenoxy7phenyl} -1,3,5-triazin-2,4,6(1H,3H,5H)-trione, Verfahren zu ihrer Herstellung und ihre pharmazeutische Verwendung als Coccidiosemittel für verschiedene Arten von Tieren, gegebenenfalls auch für den Menschen.

Gemäß der Erfindung werden 1- { 4-/4-(Fluoralkylmethyl-thio-, -sulfinyl- oder -sulfonyl)phenoxy7phenyl } 1,3,5-triazin-2,4,6(1H,3H,5H)-trione der Formel

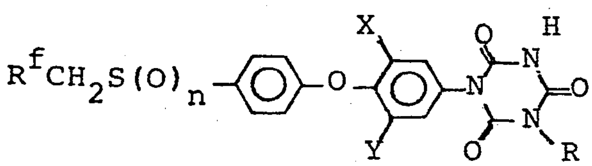

(I),

worin

$R^f$ für Fluoralkyl, z.B. Trifluormethyl und Tetrafluorethyl steht, X und Y gleich oder verschieden sind und jeweils für Wasserstoff, niederes Alkyl, niederes Alkoxy oder Halogen stehen, R für Methyl, Ethyl oder Propyl steht und n eine ganze Zahl von 0, 1 oder 2 ist, und ihre pharmazeutisch unbedenklichen Salze hergestellt. Diese Verbindungen sind wertvoll aufgrund ihrer Aktivität gegen die Coccidiose beim Menschen und verschiedenen Arten von Tieren.

Die Verbindungen der Formel (I) und ihre Salze können hergestellt werden durch

A) Umsetzung eines Harnstoffs der Formel

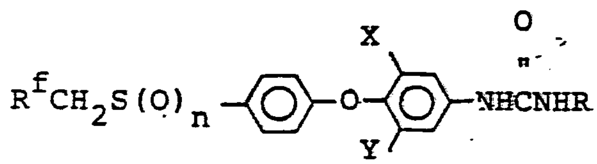

(II),

worin $R^f$, X, Y, R und n die vorstehend genannten Bedeutungen haben, mit einem Carbonylisocyanat der Formel

$$R^2-\overset{O}{\overset{\|}{C}}-NCO \quad \text{(III)}$$

worin $R^2$ für Halogen, Alkoxy oder Aryloxy steht und, im Falle der Salze, Umsetzung einer Verbindung der Formel (I) mit einer geeigneten salzbildenden Komponente oder

b) Umsetzung einer Verbindung der Formel (I),

worin $R^f$, X, Y und R die vorstehend genannte Bedeutung haben und n für Null steht, mit einem Oxidationsmittel. Bei Verwendung von Wasserstoffperoxid als Oxidationsmittel kann der Ablauf der Reaktion durch die folgende Gleichung dargestellt werden:

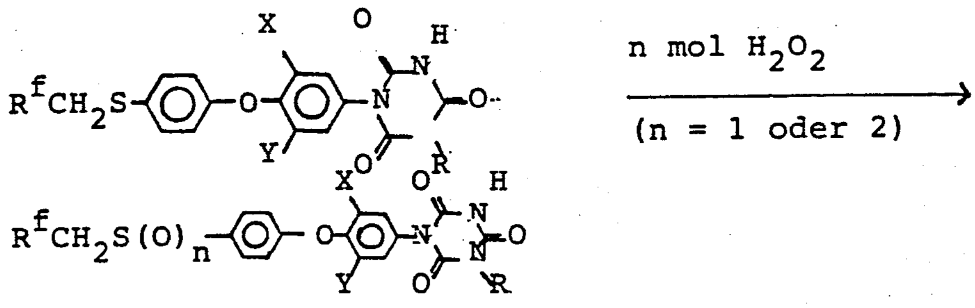


Die meisten substituierten Harnstoffe der Formel (II), die als Ausgangsmaterialien verwendet werden, waren bisher unbekannt, können jedoch leicht nach an sich bekannten Verfahren hergestellt werden, nämlich durch

A) Umsetzung von 4-[4-(Fluoralkylmethylthio-, -sulfinyl- oder -sulfonyl-)phenoxy]anilinen mit substituierten Isocyanaten in einem inerten Lösungsmittel bei Temperaturen zwischen 0°C und 130°C oder

B) durch Umsetzung von 4-[4-(Fluoralkylmethylthio-, -sulfinyl- oder -sulfonyl-)phenoxy]phenylisocyanaten mit Alkylaminen in einem inerten Lösungsmittel bei Temperaturen zwischen 0°C und 100°C.

Veröffentlicht wird die alternative Herstellung von Harnstoffen in "Methoden der org. Chemie", (Houben-Weyl), 4. Auflage, Band III, Seite 157 und 158. Als Verdünnungsmittel für die Umsetzung der Harnstoffe der Formel (II) mit Carbonylisocyanaten der Formel (III) eignen sich sämtliche organischen Lösungsmittel, die in dieser Reaktion inert sind. Hierzu gehören außer Pyridin vorzugsweise aromatische Kohlenwasserstoffe, z.B. Benzol, Toluol und Xylol, halogenierte Kohlenwasserstoffe, z.B. Dichlorethan und Tetrachlormethan, halogenierte aromatische Kohlenwasserstoffe, z.B. Chlorbenzol und Dichlorbenzol, und Ether, z.B. Tetrahydrofuran und Dioxan. Die Temperatur der Umsetzung der Harnstoffe der Formel (II) mit Carbonylisocyanaten der Formel (III) kann innerhalb eines weiten Bereichs variiert werden. Im allgemeinen wird die Reaktion bei einer Temperatur zwischen 0°C und 150°C, vorzugsweise zwischen 20°C und 100°C durchgeführt.

In der vorstehend genannten Reaktionsstufe kann die Reaktion unter Normaldruck oder unter erhöhtem Druck durchgeführt werden. Im allgmeeinen wird die Reaktion unter Normaldruck durchgeführt. Mögliche Oxidationsmittel für die Umwandlung der Fluoralkylmethylthioverbindungen der Formel (I), in der n für Null steht, in die entsprechenden oxidierten Verbindungen der Formel (I), in der n für 1 oder 2 steht, sind beispielsweise Wasserstoffperoxid, Persäuren, z.B. m-Chlorperbenzoesäure, Chromsäure, Kaliumpermanganat und Natriumperiodat.

Eine erhaltene Verbindung kann beispielsweise durch ihre Umsetzung mit einer anorganischen oder organischen Base in ein entsprechendes Salz umgewandelt werden.

Die entsprechenden Aniline der Formel

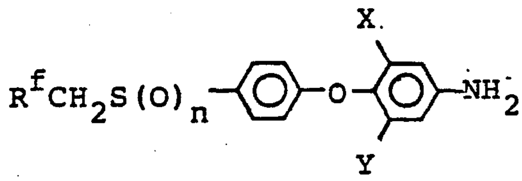

(IV)

können hergestellt werden durch Reduktion der entsprechenden Nitroverbindungen der Formel

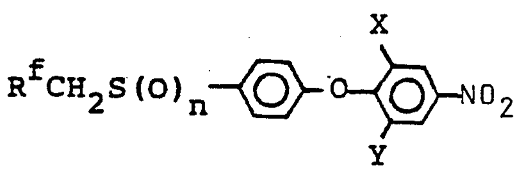

(V)

Die Nitroverbindungen der Formel (V) können durch die Kondensation einer Verbindung der Formel

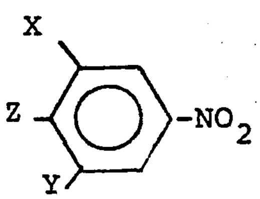

(VI)

worin Z ein Halogenatom, z.B. Chlor oder Brom, ist, mit 4-(Fluoralkylmethylthio-, -sulfinyl- oder -sulfonyl)-phenol der Formel

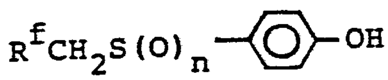 (VII)

hergestellt werden.

In allen Verbindungen der Formel (IV), (V), (VI) und (VII) haben $R^f$, X, Y und n die vorstehend genannten Bedeutungen.

Die Phenolverbindungen der Formel (VII), in der n für Null steht, können hergestellt werden durch Umsetzung von 4-Hydroxythiophenol mit Fluoralkylmethylsulfonat der Formel

$$R^2\text{-}SO_2\text{-}OCH_2R^f \qquad \text{(VIII)}$$

worin $R^f$ die vorstehend genannte Bedeutung hat und $R^2$ ein Kohlenwasserstoffrest, z.B. Methyl, Ethyl, Phenyl oder Tolyl, ist, in einem aprotischen polaren Lösungsmittel, z.B. N,N-Dimethylformamid oder Dimethylsulfoxid, in Gegenwart einer organischen oder anorganischen Base, z.B. Kaliumcarbonat, Natriumcarbonat, Kaliumhydroxid, Natriumhydroxid oder Natriumhydrid.

Das Fluoralkylsulfonat der Formel (VIII) kann nach Verfahren hergestellt werden, die aus Veröffentlichungen, beispielsweise Journal of the American Chemical Society, Vol. 75, S. 5978, und Journal of Organic Chemistry,

Vol. 35, S. 3195 bekannt sind, jedoch ist jede Isolierungsmethode von Fluoralkylsulfonaten nach den in den Veröffentlichungen genannten Verfahren verhältnismäßig kompliziert. Wenn eine wäßrige Natriumhydroxidlösung tropfenweise zu einem Gemisch von Fluoralkanol, z.B. ß,ß,ß-Trifluorethanol, Sulfonylchlorid, z.B. p-Toluolsulfonylchlorid, einer katalytischen Menge eines Phasenübergangskatalysators, z.B. Triethylbenzylammoniumchlorid, und eines inerten Verdünnungsmittels, z.B. Toluol, gegeben wird, verläuft die Reaktion schnell und quantitativ.

Die Temperatur der Reaktion kann innerhalb eines weiten Bereichs, vorzugsweise zwischen 20°C und 70°C variiert werden. Die Menge jedes Reaktionsteilnehmers kann stöchiometrisch sein, oder die Menge des Fluoralkanols und/oder des Natriumhydroxids kann im Überschuß vorliegen.

Die Verbindungen gemäß der Erfindung und ihre pharmazeutisch unbedenklichen, ungiftigen Salze weisen gute Aktivität gegen Arten von Coccidien bei Geflügel, z.B. Eimeria tenella (Blinddarm-Coccidiose bei Hühnern), E. Acervulina, E. brunetti, E. maxima, E. mitis, E. necatrix und E. praecox (Dünndarmcoccidiose bei Hühnern), auf.

Die Zubereitungen können außerdem zur Prophylaxe und Behandlung von Coccidiose-Infektionen anderer Arten von Hausgeflügel verwendet werden. Darüber hinaus

weisen die Verbindungen gemäß der Erfindung starke Aktivitäten bei Coccidieninfektionen von Säugern auf. Ferner können die Verbindungen bei der Behandlung oder Prophylaxe der Toxoplasmose sowohl für die Behandlung von Katzen, die für die Ausscheidung in den infektiösen Stadien (Oozysten) verantwortlich sind, und für die Behandlung von infizierten Menschen verwendet werden. Coccidieninfektionen können zu schweren Verlusten unter den Haustieren führen und stellen ein wirkliches Problem bei der Aufzucht von Geflügel und Säugetieren wie Rindvieh, Schaf, Kaninchen und Hunden, dar. Die Wirkung der bekannten Mittel gegen Coccidiose ist in den meisten Fällen auf einige wenige Arten von Geflügel begrenzt. Die Behandlung und Prophylaxe der Coccidiose bei Säugetieren stellt bisher ein großes ungelöstes Problem dar.

Die Arzneimittelzubereitungen gemäß der Erfindung enthalten eine größere oder geringere Menge, z.B. 0,1 % bis 99,5 %, vorzugsweise 0,5 % bis 90 %, wenigstens eines 1-(Fluoralkylmethylthio- oder -sulfinyl- oder -sulfonyl)-phenylsubstituierten 1,3,5-Triazins gemäß obiger Definition in Kombination mit einem pharmazeutisch unbedenklichen, ungiftigen inerten Streckmittel oder Träger, wobei der Träger ein oder mehrere feste, halbfeste oder flüssige Verdünnungs- oder Streckmittel, Füllstoffe und Zubereitungsadjuvantien enthält, die ungiftig, inert und pharmazeutisch unbedenklich sind. Diese Arzneimittelzubereitungen liegen vorzugsweise in Form von Dosierungseinheiten, d.h. gesonderten Einheiten vor, die eine vorbestimmte Menge des Medikaments enthalten, die einen Bruchteil oder einem Vielfachen der Dosis entspricht, die laut Berechnung die gewünschte therapeutische Wirkung hervorbringt. Die Dosierungseinheiten können eine, zwei, drei, vier oder mehr Einzeldosen oder als Alternative eine Hälfte, ein Drittel oder Viertel einer Einzeldosis enthalten. Eine Einzeldosis enthält vorzugsweise eine Menge, die genügt, um die gewünschte therapeutische Wirkung nach der Verabreichung bei einmaliger Anwendung einer oder mehrerer Dosierungseinheiten gemäß einer vorbestimmten Dosierungsbehandlungsvorschrift, gewöhnlich einer ganzen, halben, einem Drittel oder Viertel der einmal, zweimal, dreimal oder viermal täglich verabreichten Tagesdosis hervorzubringen. Andere therapeutische Mittel können ebenfalls vorhanden sein.

Die orale Verabreichung kann unter Verwendung von festen und flüssigen Dosierungseinheitsformen, z.B. Pulver, Tabletten, Dragees, Kapseln, Granulat, Suspensionen, Lösungen und dergleichen erfolgen.

Pulver werden hergestellt durch Mahlen der Verbindung auf eine geeignete feine Größe und Vermischen mit einnem in ähnlicher Weise gemahlenen pharmazeutischen Träger, z.B. einem eßbaren Kohlenhydrat, z.B. Stärke, Lactose, Saccharose, Glucose oder Mannit. Süßungsmittel, Aromatisierungsmittel, Konservierungsmittel, Dispergierungs- und Färbemittel können ebenfalls vorhanden sein.

Kapseln werden hergestellt durch Zubereitung eines Pulvergemisches, wie es vorstehend beschrieben wurde, und Einfüllen in geformte Gelatinehüllen. Gleitmittel und Schmiermittel, z.B. kolloidales Siliciumdioxid, Talkum, Magnesiumstearat, Calciumstearat oder festes Polyethylenglykol, können dem Pulvergemisch vor dem Füllvorgang zugesetzt werden. Ein Sprengmittel oder löslichmachendes Mittel, z.B. Agar-Agar, Calciumcarbonat oder Natriumcarbonat, können ebenfalls zugesetzt werden, um die Verfügbarkeit des Medikaments zu verbessern, wenn die Kapsel eingenommen wird.

Tabletten werden beispielsweise hergestellt durch Herstellung eines Pulvergemisches, Granulieren oder Verperlen, Zusetzen eines Gleitmittels und Sprengmittels und Pressen zu Tabletten. Ein Pulvergemisch wird hergestellt durch Mischen der in geeigneter Weise zerkleinerten Verbindung mit einem Verdünnungs- oder Streckmittel oder einer Base in der vorstehend beschriebenen Weise und, falls gewünscht, mit einem Bindemittel wie Carboxymethylcellulose, einem Alginat, Gelatine oder Polyvinylpyrrolidon, einem Lösungsverzögerer, z.B. Paraffin, einem Resorptionsbeschleuniger, z.B. einem quaternären Salz, und/oder einem Adsorptionsmittel wie Bentonit, Kaolin oder Dicalciumphosphat. Das Pulvergemisch kann granuliert werden durch Benetzen mit einem Bindemittel wie Sirup, Stärkepaste, Schleim von Gummi arabicum oder Lösungen von Cellulosematerialien oder polymeren Materialien, und Pressen

durch ein Sieb. Als Alternative des Granulierens kann das Pulvergemisch durch die Tablettiermaschine gegeben werden, und die hierbei erhaltenen unvollkommen geformten Perlen können zu Granulat zerbrochen werden. Das Granulat kann zur Verhinderung des Anklebens an die Tablettenbildungsdüsen durch Zusatz von Stearinsäure, einem Stearatsalz, Talkum oder Mineralöl geschmiert werden. Das geschmierte Gemisch wird dann zu Tabletten gepreßt. Die Medikamente können auch mit rieselfähigen inerten Trägern kombiniert und direkt zu Tabletten gepreßt werden, ohne daß sie die Granulier- oder Verperlungsstufen durchlaufen. Ein klarer oder undurchsichtiger Schutzüberzug, bestehend aus einem abdichtenden Überzug aus Schellack, ein Überzug aus Zucker oder polymerem Material und ein polierender Wachsüberzug können vorgesehen werden. Diesen Überzügen können Farbstoffe zur Unterscheidung verschiedener Einheitsdosierungen zugesetzt werden.

Orale Flüssigkeiten, z.B. Lösungen, Sirupe und Elixiere, können in Dosierungseinheitsform so hergestellt werden, daß eine gegebene Menge eine vorbestimmte Menge der Verbindung enthält. Sirupe können durch Auflösen der Verbindung in einer in geeigneter Weise schmackhaft gemachten wäßrigen Saccharoselösung hergestellt werden, während Elixiere durch Verwendung eines ungiftigen alkoholischen Trägers hergestellt werden. Suspensionen können durch Dispergieren der Verbindung in einem ungiftigen Träger hergestellt werden. Löslichmachende Mittel und Emulgatoren, z.B. ethoxylierte Isostearylalkohole und Polyoxyethylensorbitester, Konservierungsmittel, Geschmack verleihende Zusatzstoffe, z.B. Pfefferminzöl und Saccharin und dergleichen können ebenfalls zugesetzt werden.

Falls geeignet oder angemessen, können Dosierungseinheitszubereitungen für die orale Verabreichung in Form von Mikrokapseln hergestellt werden. Die Zubereitung kann auch so hergestellt werden, daß die Freigabe verlängert oder in Gang gehalten wird, beispielsweise durch Umhüllen oder Einbetten des feinteiligen Materials in Polymerisaten, Wachs oder dergleichen.

Die parenterale Verabreichung kann unter Verwendung von flüssigen Dosierungseinheitsformen, z.B. sterilen Lösungen und Suspensionen, die für die subkutane, intramuskuläre oder intravenöse Injektion vorgesehen sind, erfolgen. Diese Zubereitungen werden hergestellt durch Suspendieren oder Auflösen einer gemessenen Menge der Verbindung in einem für die Injektion geeigneten ungiftigen flüssigen Träger, z.B. einem wäßrigen oder öligen Medium, und Sterilisation der Suspension oder Lösung. Als Alternative wird eine abgemessene Menge der Verbindung in eine Phiolenflasche gegeben, und die Phiolenflasche und ihr Inhalt werden sterilisiert und zugeschmolzen. Eine zugehörige Phiolenflasche oder ein zugehöriger Träger können zum Vermischen vor der Verabreichung vorgesehen werden. Ungiftige Salze und Salzlösungen können zugesetzt werden, um die Injektionslösung

isotonisch zu machen. Stabilisatoren, Konservierungsmittel und Emulgatoren können ebenfalls zugesetzt werden.

Die rektale Verabreichung kann bei Verwendung von Suppositorien wirksam sein, in denen die Verbindung mit niedrigschmelzenden, wasserlöslichen oder unlöslichen Feststoffen, z.B. Polyethylenglykol, Kakaobutter, höheren Estern, z.B. Myristylpalmitat oder deren Gemischen gemischt ist.

Die örtliche Anwendung kann unter Verwendung von festen Dosierungseinheitsformen, z.B. Pulvern, oder flüssigen oder halbflüssigen Dosierungseinheitsformen, z.B. Lösungen, Suspensionen, Salben, Pasten, Cremes und Gelen erfolgen. Die Pulver werden unter Verwendung von Trägern wie Talkum, Bentonit, Kieselsäure, Polyamidpulver und dergleichen formuliert werden. Den flüssigen und halbflüssigen Zubereitungen können Träger zusätzlich zu den vorstehend beschriebenen, z.B. Polyethylenglykol, Pflanzen- und Mineralöle, Alkohole, z.B. Isopropanol, zugesetzt werden. Andere Hilfsstoffe, z.B. Emulgatoren, Konservierungsmittel, färbende Mittel, Duftstoffe und dergleichen, können ebenfalls anwesend sein. Die Zubereitungen können auch als Aerosole verabreicht werden, wobei die üblichen Treibmittel, z.B. die Chlorfluorkohlenwasserstoffe, verwendet werden.

Die bevorzugte Tagesdosis beträgt 25 mg bis 25 g des aktiven Ingrediens.

Während zu den Darreichungswegen die orale, parenterale (d.h. intramuskuläre, intraperitoneale und intravenöse) rektale und örtliche Verabreichung gehört, wird die orale Verabreichung besonders bevorzugt.

Die Erfindung umfaßt ferner Futter- und Nahrungsmittel, die 25 bis 5000, vorzugsweise 50 bis 250 ppm einer Verbindung gemäß der Erfindung in Kombination mit einem geeigneten eßbaren Material, z.B. mit dem in der folgenden Tabelle beschriebenen Hühnerfuttermittel enthalten:

| | |
|---|---|
| 52,000 % | geschrotetes Futtergetreide |
| 17,995 % | geschrotete Sojabohnen |
| 5,000 % | Vollweizenmehl |
| 3,000 % | Fischmehl |
| 3,000 % | Tapiokamehl |
| 3,000 % | Mehl der grünen Luzerne |
| 2,000 % | gemahlene Weizenkeime |
| 2,000 % | Sojaöl |
| 1,600 % | Fischgrätenmehl |
| 1,500 % | Molkepulver |
| 1,400 % | Calciumcarbonat für Futter- und Lebensmittel |
| 1,000 % | Calciumphosphat für Futter- und Lebensmittel |
| 1,000 % | Melasse |
| 0,500 % | Bierhefe |
| 0,005 % | 1-[3,5-Dichlor-4-(4'(ß,ß,ß-trifluorethylthio)-phenoxy)-phenyl]-3-methyl-1,3,5-triazin-2,4,6-(1H,3H,5H)-trion |
| 100,000 % | |

Ein solches Futter- und Nahrungsmittel kann sowohl für Heilzwecke als auch für prophylaktische Zwecke verwendet werden.

Die Erfindung umfaßt ferner ein Konzentrat oder eine Vormischung, die 1 bis 30 %, vorzugsweise 10 bis 20 Gew.-% einer Verbindung gemäß der Erfindung in Mischung mit einem eßbaren organischen oder anorganischen Träger enthält, z.B. Maismehl oder Mais- und Sojabohnenmehl oder Mineralsalze, die vorzugsweise eine geringe Menge eines eßbaren Staubverhütungsöls, z.B. Maisöl oder Sojaöl, enthalten. Die hierbei erhaltene Vormischung kann dann dem vollständigen Geflügelfuttermittel vor seiner Verfütterung an die Tiere zugesetzt werden.

Die Verbindungen gemäß der Erfindung können auch mit dem Trinkwasser für die Tiere zur Massenbehandlung oder -prophylaxe der Coccidiose gemischt werden.

Das Futtermittel kann sowohl für Heilzwecke als auch für prophylaktische Zwecke verwendet werden.

Für die Heilung und Prophylaxe der Coccidiose bei Geflügel, insbesondere bei Hühnern, Enten, Gänsen und Truthähnen, werden 25 bis 100 ppm, vorzugsweise 50 bis 100 ppm einer Verbindung gemäß der Erfindung mit einem geeigneten eßbaren Material, z.B. einem nahrhaften Futtermittel, gemischt. Falls gewünscht, können diese Mengen erhöht werden, besonders wenn die Verbindung vom Empfänger gut vertragen wird.

Für die Behandlung von Einzeltieren, z.B. im Falle der Behandlung der Coccidiose bei Säugetieren oder der Toxoplasmose, werden vorzugsweise Mengen von 5 bis 250 mg/kg Körpergewicht täglich verabreicht, um die gewünschten wirksamen Ergebnisse zu erzielen. Trotzdem kann es zeitweilig notwendig sein, von den genannten Mengen abzuweichen, insbesondere in Abhängigkeit vom Körpergewicht des Versuchstieres oder der Art der Verabreichungsmethode, aber auch wegen der Tiergattung und seiner individuellen Reaktion auf das Medikament oder der Art der Formulierung und der Zeit oder dem Abstand, zu dem es verabreicht wird. So kann es in gewissen Fällen genügen, mit weniger als der vorstehend genannten Mindestmenge auszukommen, während in anderen Fällen die genannte obere Grenze überschritten werden muß. Bei der Verabreichung größerer Mengen kann es zweckmäßig sein, diese im Verlauf des Tages in mehrere Einzeldarreichungen zu unterteilen.

Die coccidiostatische Aktivität von Verbindungen, die für die Verbindungen gemäß der Erfindung repräsentativ sind, wird in Tabelle 1 veranschaulicht, wo Eimeria tenella (Blinddarm-Coccidiose/Hühner), Eimeria acervulina und Eimeria maxima (Dünndarm-Coccidiose von Hühnern) als Beispiele der Aktivität im Falle der Coddidiose bei Geflügel genannt sind, während Eimeria falciformus (Coccidiose/Mäuse) als Coccidium bei Säugetieren angegeben ist.

Wenn beispielsweise 9 bis 11 Tage alte Küken mit 4000 sporulierten Oozysten von stark virulenten Stämmen von E. acervulina, E. maxima und E. tenella, den Krankheitserregern der intestinalen Coccidiose infiziert werden, scheiden die unbehandelten Vergleichstiere täglich 300,000 bis 500,000 Oozysten/g Kot vom fünften bis neunten Tage nach der Infektion aus. Im Verlauf der Krankheit ist die Gewichtszunahme wesentlich verringert, und ernsthafte, makroskopisch nachweisbare pathologische Veränderungen treten hauptsächlich in den Därmen auf, was zu ernsthaften hämorrhagischen Diarrhoeen führen kann, und die Küken können sterben. Bei der Untersuchung der Wirksamkeit gegen E. acervulina, E. maxima und E. tenella wurden die Verbindungen gemäß der Erfindung 3 Tage vor der Infektion bis 8 Tage nach der Infektion (Ende des Versuchs) verabreicht.

Die Zahl der Oozysten wurde mit Hilfe der McMaster-Kammer bestimmt (siehe Engelbrecht und Mitarbeiter "Parasitologische Arbeitsmethoden in Medizin und Veterinärmedizin", S. 172, Akademie-Verlag, Berlin (1965)).

Die Behandlung der Infektion mit Eimeria falciformis bei Mäusen, die als Beispiel der Coccidiose bei Säugetieren erwähnt ist, fand am ersten, zweiten, dritten, sechsten, siebten und achten Tag nach der Infektion statt. Die Infektion erfolgte mit 10,000 sporulierten Oozysten pro Maus (Gewicht 15 g). Im Falle der unbehandelten Vergleichstiere fanden massive Ausscheidung von Oozysten, blutiger Durchfall und 30 % Sterblichkeit der Tiere, die der Infektion zuzuschreiben waren, vom siebten Tage nach der Infektion an statt.

Als wirksam wurden diejenigen Dosen angesehen, die die Ausscheidung von Oozysten und/oder klinische Symptome der Coccidiose einschließlich der Mortalität vollständig oder in hohem Maße verhüteten.

Die niedrigsten wirksamen Dosierungen, die unter den Versuchsbedingungen gefunden wurden, waren niedriger als diejenigen der anticoccidialen Medikamente, die gewöhnlich zur Verhütung der Coccidiose bei Geflügel verwendet werden. Ferner sind die Verbindungen gemäß der Erfindung - außer einem günstigen Verhalten hinsichtlich der Rückstände im Körper - sehr wirksam gegen die Coccidiose bei Säugetieren (Tab. 1).

Die weit verbreitete Resistenz der Coccidiose gegen Coccidiosemittel erfordert wirksamere Verbindungen, die von neuen Klassen von chemischen Verbindungen abgeleitet sind. Auch von diesem Standpunkt aus bereichern die Verbindungen gemäß der Erfindung in wertvoller Weise die Vielfalt der Medikamente.

Tabelle 1

Vergleich der wirksamen Mindestdosierungen von Verbindungen gemäß der Erfindung mit denjenigen von zwei üblicherweise in der Praxis verwendeten Coccidiosemitteln

| | E. acervulina ppm | E. maxima ppm | E. tenella ppm | E. falciformis mg/kg |
|---|---|---|---|---|
| 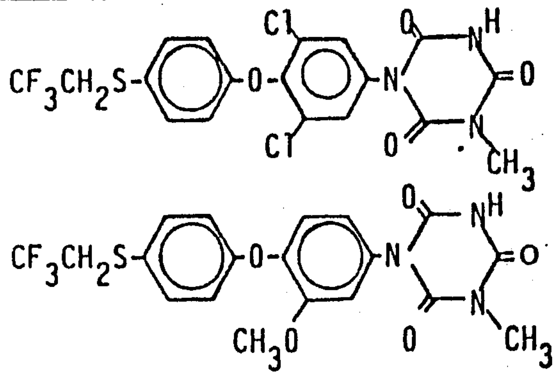 | 25 | 25 | 50 | 1 |
| | 50 | 50 | 50 | 25 |
| 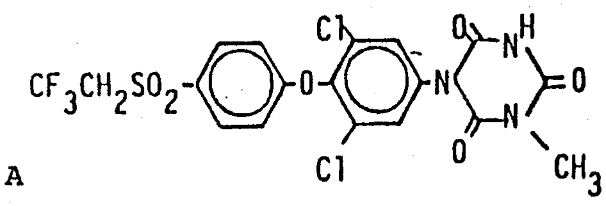 | 10 | 10 | 10 | 100 |
| A | 125 | 125 | 125 | 500 |
| M | 50 | 50 | 75 | 250 |

A = 1/[(4-Amino-2-propyl-5-pyrimidinyl)-methyl]-2-picoliniumchloridhydrochlorid

M = 2-(5-Ethyltetrahydro-5-(tetrahydro-3-methyl-5-(tetrahydro-6-hydroxy-6(hydroxyethyl)-3,5-dimethylpyran-2-yl)-2-furyl)-2-furyl)-9-hydroxy-ß-methoxy-αγ,2,8-tetramethyl-1,6-dioxaspiro-(4,5)decan-7-buttersäure

Herstellungsbeispiel 1

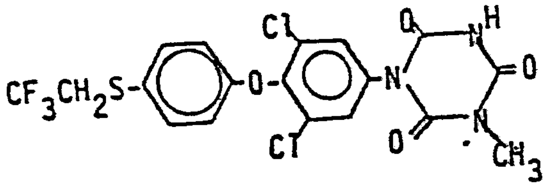

Zu einer Lösung von 4,25 g N-{3,5-Dichlor-4-[4-(ß,ß,ß-trifluorethylthio)phenoxy]phenyl}-N'-methylharnstoff in 100 ml Toluol wird unter Rühren tropfenweise eine Lösung von 1,16 g Chlorcarbonylisocyanat in 2 ml Toluol gegeben. Das Gemisch wird 1 Stunde bei 20 bis 30°C gerührt und dann 3 Stunden gekocht. Nach der Abkühlung wird das Reaktionsgemisch im Vakuum eingedampft. Der hierbei erhaltene Feststoff wird aus Ethanol umkristallisiert, wobei 3,8 g 1-{3,5-Dichlor-4-[4-(ß,ß,ß-trifluorethylthio)-phenoxy]phenyl}-3-methyl-1,3,5-triazin-2,4,6-(1H,3H,5H)-trion vom Schmelzpunkt 212 bis 213°C erhalten werden.

Die folgenden Verbindungen der Formel (I) werden nach dem gleichen Verfahren wie es im Herstellungsbeispiel 1 beschrieben wurde, hergestellt:

1a) 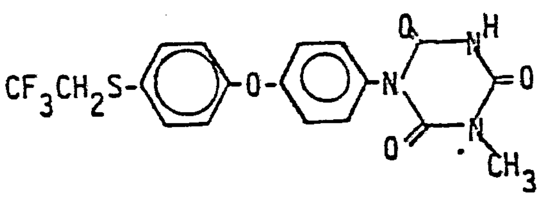 Schmelzp. 141 - 143°C

1b) 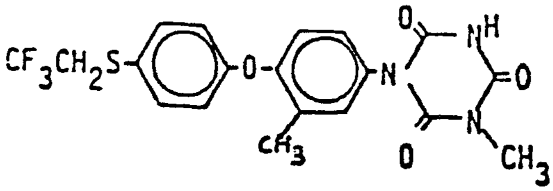 Schmelzp. 175 - 177°C

1c) 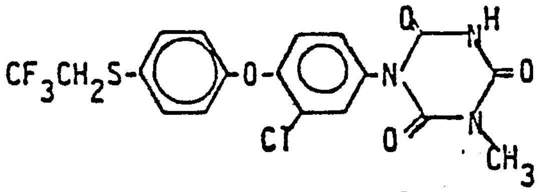 Schmelzp. 167 - 178°C

1d) 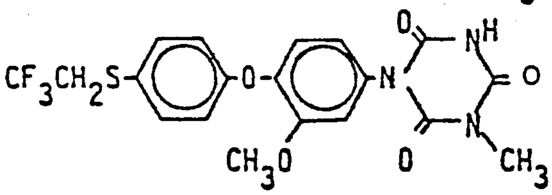 Schmelzp. 187 - 189°C

1e) 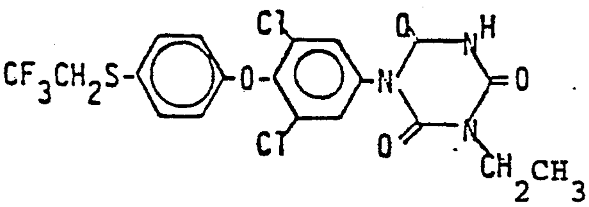 Schmelzp. 183 - 185°C

1f) 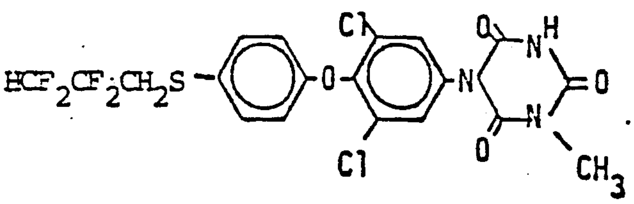 Schmelzp. 184 - 185°C

1g) 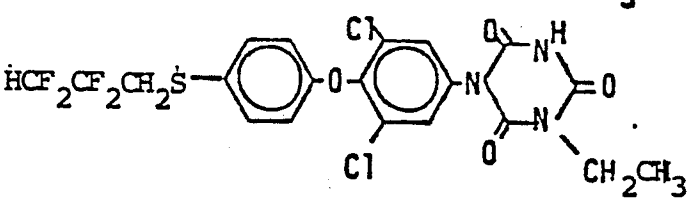 Schmelzp. 105 - 112°C

1h) 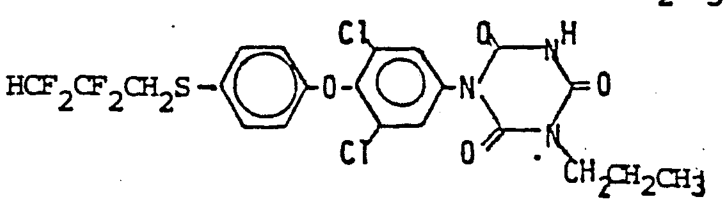 Schmelzp. 150 - 157°C

Herstellungsbeispiel 2

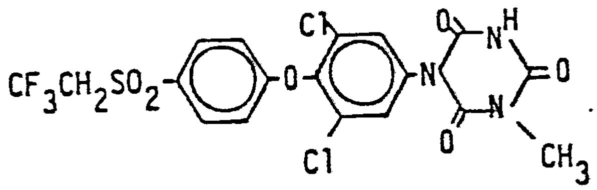

Zu einer Lösung von 3,8 g der in der vorstehend beschriebenen Weise hergestellten Verbindung 1 in 100 ml Eisessig werden unter Rühren tropfenweise bei einer Temperatur von etwa 20°C 10 g 30 %ige wäßrige Wasserstoffperoxidlösung gegeben. Das Gemisch wird 1 Stunde bei 20 bis 30°C gerührt und dann 2 Stunden gekocht.

Zum gekühlten Reaktionsgemisch wird eine Lösung von 10 g Natriumthiosulfat in 100 ml Wasser gegeben und das Ganze wird 0,5 Stunden bei Raumtemperatur gerührt, unter vermindertem Druck eingedampft und dann mit 20 ml Wasser gut gemischt. Der hierbei erhaltene Feststoff wird abfiltriert, mit Wasser gewaschen, getrocknet und aus Ethanol umkristallisiert, wobei 2,5 g 1- {3,5-Dichlor-4-[4-(ß,ß,ß-trifluorethylsulfonyl)-phenoxy]-phenyl} -3-methyl-1,3,5-triazin-2,4,6(1H,3H,5H)-trion vom Schmelzpunkt 269 bis 272°C erhalten werden.

Herstellungsbeispiel 3

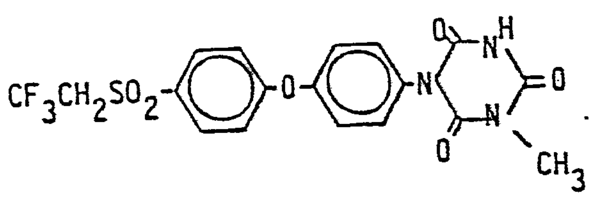

Zu einer Suspension von 1,9 g N-{4-/4-(ß,ß,ß-Trifluorethylsulfonyl)phenoxy7phenyl}-N'-methylharnstoff in 10 ml 1,2-Dichlorethan wird unter Rühren eine Lösung von 0,6 g Chlorcarbonylisocyanat in 2 ml 1,2-Dichlorethan gegeben. Das Gemisch wird 5 Stunden gekocht und dann auf Raumtemperatur gekühlt. Der nach dem Eindampfen des Reaktionsgemisches erhaltene Feststoff wird aus Ethanol umkristallisiert, wobei 1,6 g 1-{4-/4-(ß,ß,ß-Trifluorethylsulfonyl)phenoxy7phenyl}-3-methyl-1,3,5-triazin-2,4,6(1H,3H,5H)-trion vom Schmelzpunkt 209 bis 213°C erhalten werden.

Patentansprüche

1. 1- {4-[4-(Fluoralkylmethylthio-, -sulfinyl- oder -sulfonyl-)phenoxy]phenyl} -1,3,5-triazin-2,4,6-(1H,3H,5H)-trione der Formel

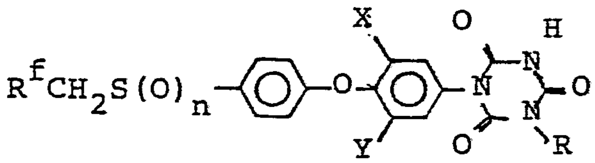

worin

$R^f$ für Fluoralkyl steht und

X und Y gleich oder verschieden sind und für Wasserstoff, niederes Alkyl, niederes Alkoxy oder Halogen stehen,

R ein niederer Alkylrest und

n eine ganze Zahl von 0, 1 oder 2 ist, oder ihre physiologisch unbedenklichen Salze.

2. Verbindungen nach Anspruch 1, ausgewählt aus

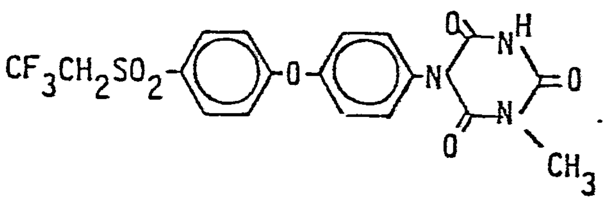

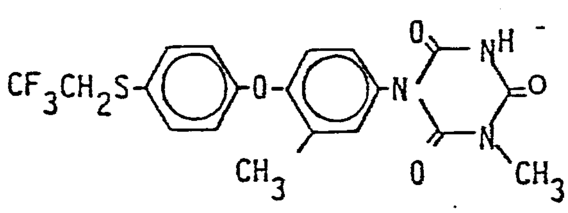

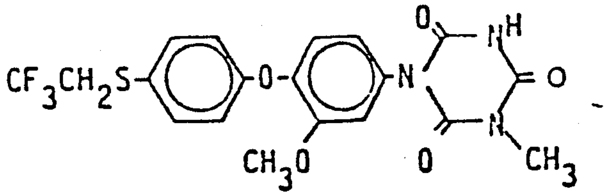

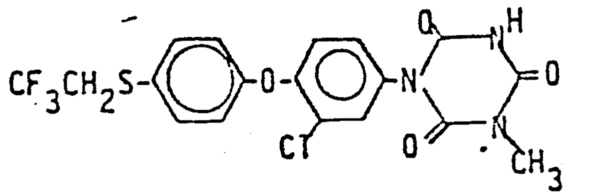

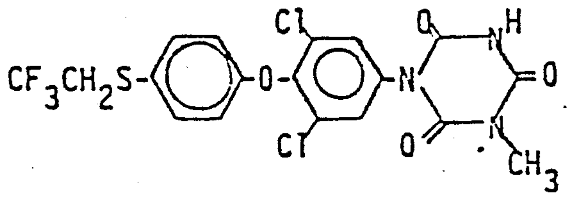

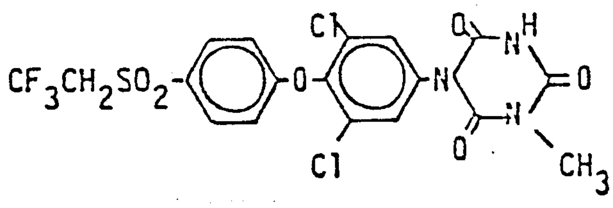

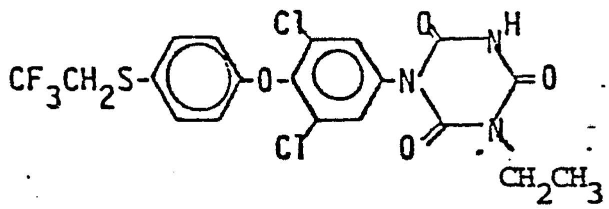

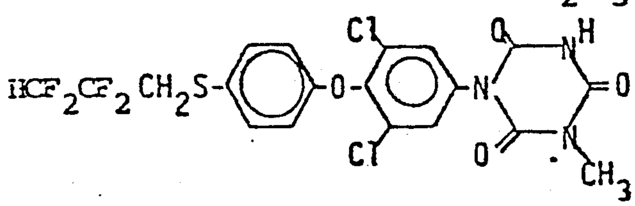

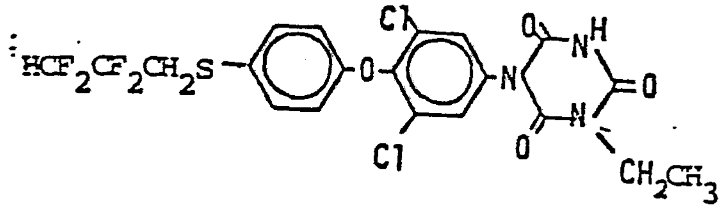

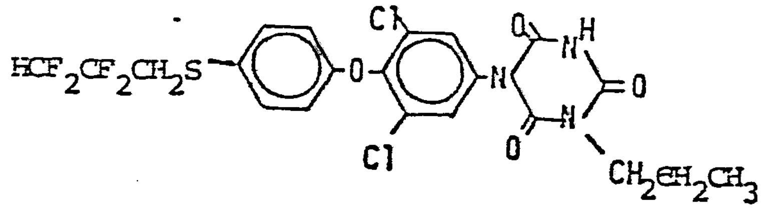

3. Verfahren zur Herstellung von Verbindungen nach Anspruch 1 und 2, dadurch gekennzeichnet, daß man

a) einen Harnstoff der Formel

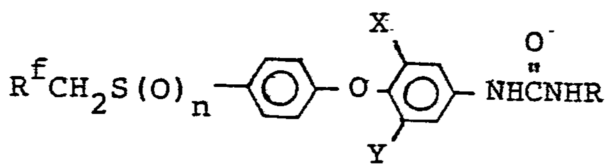

(II)

worin $R^f$,X,Y,R und n die vorstehend genannten Bedeutungen haben, mit einem Carbonyliso-cyanat der Formel

$$R^2-\overset{O}{\overset{\parallel}{C}}-NCO \qquad (III),$$

worin $R^2$ für Halogen, Alkoxy oder Aryloxy steht und, im Falle der Salze, Umsetzung einer Verbindung der Formel (I) mit einer geeigneten salzbildenden Komponente oder

b) eine Verbindung der Formel (I), in der $R^f$, X, Y und R die vorstehend genannten Bedeutungen haben und n für Null steht, mit einem Oxidationsmittel umsetzt.

4. Coccidiose-Mittel, enthaltend wenigstens eine Verbindung nach Anspruch 1 und 2 zusammen mit üblichen Trägern und Hilfsstoffen.

5. Verwendung der Verbindungen nach Anspruch 1 und 2 bei der Bekämpfung der Coccidiose.

6. Verwendung von Verbindungen der allgemeinen Formel (I) in Anspruch 1 als Zusatz zu Futter- und Nahrungsmitteln in der Tierernährung.

7. Verwendung von Verbindungen der allgemeinen Formel (I) in Anspruch 1 in Konzentrationen von 25 bis 100 ppm.

8. Tiernahrung und in der Tierhaltung verwendete Trinkwasser, enthaltend Verbindungen der allgemeinen Formel (I) in Anspruch 1.

9. Verfahren zur Herstellung von Tiernahrung und/oder in der Tierhaltung verwendeten Trinkwassern, dadurch gekennzeichnet, daß man der Tiernahrung und/oder den Trinkwassern der allgemeinen Formel (I) in Anspruch 1 zusetzt.

10. Praemixe für die Tiernahrung und/oder in der Tierhaltung verwendete Trinkwasser, enthaltend Verbindungen der allgemeinen Formel (I) in Anspruch 1.

Europäisches Patentamt

# EUROPÄISCHER RECHERCHENBERICHT

0123217
Nummer der Anmeldung

EP 84 10 4094

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | FR-A-2 388 559 (BAYER AG)<br>* Ansprüche * | 1,4 | A 61 K 31/53<br>C 07 D 251/34<br>A 01 N 43/64 |
| A | NL-A-7 503 345 (BAYER AG)<br>* Seite 18, Zeile 29; Seite 20, Zeile 15 * | 1,4 | |
| A | DE-A-2 313 721 (BAYER AG)<br>* Seiten 28,33; Ansprüche 1,4,5 * | 1,4 | |

| RECHERCHIERTE SACHGEBIETE (Int. Cl. 3) |
|---|
| C 07 D 251/00 |

Der vorliegende Recherchenbericht wurde für alle Patentansprüche erstellt.

| Recherchenort | Abschlußdatum der Recherche | Prüfer |
|---|---|---|
| DEN HAAG | 17-07-1984 | VAN BIJLEN H. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument